# EUROPEAN PATENT SPECIFICATION

(11) **EP 1 808 150 B1**
(45) Date of publication and mention of the grant of the patent: **15.05.2013**
(21) Application number: 07000508.7
(22) Date of filing: 11.01.2007
(51) Int. Cl.: A61F 2/16

(54) **Intraocular lens injector and intraocular lens injecting system including the same**
Injektor für Intraokularlinsen und diesen umfassendes Implantationssystem
Injecteur pour lentille intraoculaire et système d'injection utilisant celui-ci

(30) Priority: 11.01.2006 JP 2006004216
(43) Date of publication of application: 18.07.2007
(73) Proprietor: NIDEK CO., LTD, Gamagori-shi Aichi (JP)
(72) Inventor: Nagasaka, Shinji, Hiroishi-cho Gamagori-shi Aichi (JP)
(74) Representative: Weber, Joachim

(56) References cited:
- EP-A- 1 360 944
- EP-A1- 1 481 652
- EP-A1- 1 502 559
- WO-A-03/045285
- US-A- 4 681 102

## Description

### BACKGROUND OF THE INVENTION

The present invention relates to an intraocular lens injector that injects an intraocular lens into an eye, and an intraocular lens injecting system including the intraocular lens injector.

As a type of operating instrument for operating a cataract, an intraocular lens injector, which injects an intraocular lens into an eye with a clouded crystalline lens extracted, is generally used.

Lubricant (viscoelastic substance) is injected into such the injector to move smoothly the intraocular lens in the injector. In general, after an openable and closable lens place portion of the injector is opened, the lubricant is injected into the injector.

The document W003045285 discloses an intraocular lens injector according to the preamble of claim 1, having a loading channel fillable at least partially, but preferably completely, with a viscous lubricant.

### Summary of the Invention

It is an object of the invention to provide an intraocular lens injector where lubricant is easily injected, and an intraocular lens injecting system including the intraocular lens injector.

In order to solve the above problem, the present invention is characterized by the features of claim 1.
(2) The intraocular lens injector according to claim 1, wherein
   the lens place portion includes a fixed part fixed to the inserting portion and a movable part connected to the fixed part through the hinge, and
   the lubricant is injected into a space between the fixed and movable parts in a state that the fixed and movable parts are opened.
(3) The intraocular lens injector according to (2), wherein the first injecting passage is formed in the fixed part so that an inlet is formed at an outerwall of the fixed part and an outlet is formed at a sidewall of the fixed part facing the movable part.
(4) The intraocular lens injector according to claim 1, wherein the first injecting passage includes a restricting portion for restricting movement of an injecting needle of a syringe for injecting the lubricant.

(5) An intraocular lens injecting system comprising a holder that holds an intraocular lens injector as previously described.
(6) The intraocular lens injecting system according to (5), wherein
   the holder includes a holding member that holds the intraocular lens in the open lens place portion, and
   the lubricant is injected into the open lens place portion.

### Brief Description of the Drawings

Fig. 1 is a schematic view showing an intraocular lens injecting system according to an embodiment of the invention;
Fig. 2 is a schematic view showing an intraocular lens;
Fig. 3 is a schematic view showing a lens cartridge of an injector;
Figs. 4A and 4B are schematic views showing a handpiece of the injector;
Fig. 5 is a schematic view showing a holder that holds the injector;
Figs. 6A and 6B are views showing a state in which the injector is held by the holder;
Fig. 7 is a schematic view of a syringe for injecting lubricant;
Figs. 8A, 8B, and 8C are views illustrating a configuration for injecting the lubricant into the lens cartridge;
Fig. 9 is a view showing the injector rotating to direct a hinge down; and
Figs. 10A and 10B are views illustrating a configuration that restricts movement of the end of an injecting needle.

### DETAILED DESCRIPTION OF PREFERRED EMBODIMENTS

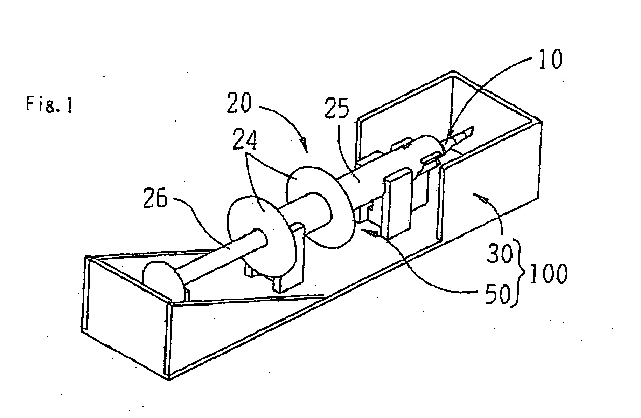

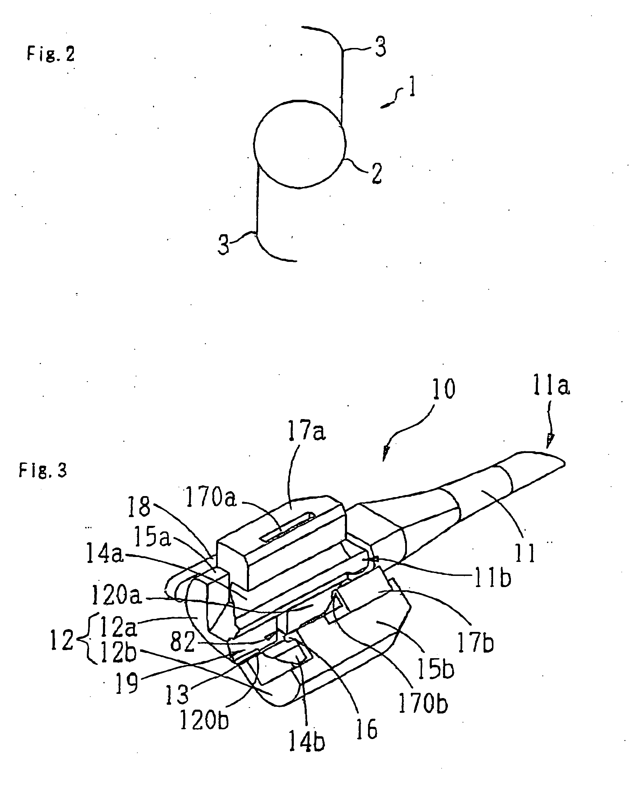

Preferred embodiments of the invention will be described hereafter with reference to accompanying drawings. Fig. 1 is a schematic view showing an intraocular lens injecting system according to an embodiment of the invention. Fig. 2 is a schematic view showing an intraocular lens. An intraocular lens injecting system 100 includes an intraocular lens injector 50 that injects a foldable intraocular lens 1 into an eye, and a holder (a casing) 30 that fixedly holds the injector 50. The injector 50 includes a lens cartridge (a lens holding unit) 10 that holds the intraocular lens 1, and a handpiece (a cartridge holding unit) 20 as an injector body that holds the cartridge 10.

The intraocular lens 1 includes an optical portion 2 having predetermined refractive power and a supporting portion 3 that supports the optical portion 2 in an eye. The optical portion 2 is formed of an existing material that is used for a foldable optical portion of an intraocular lens, such as, HEMA (Hydroxyethylmethacrylate), a composite of acrylic acid ester and methacrylate, etc. Further, the supporting portion 3 is formed of an existing material that is used for a supporting portion of an intraocular lens, such as PMMA (Polymethylmethacrylate) etc. The shape of the intraocular lens 1 is not limited to those in the embodiments of the invention and any shape may be applied as long as it is foldable.

Fig. 3 is a schematic view of the cartridge 10. Incidentally, upside and downside in the description for Fig. 3 correspond to upside and downside in a normal usage state of the injector 50 (the cartridge 10). The cartridge 10 includes an inserting portion 11 that is to be inserted through an incision on an eye and a lens place portion 12 where the intraocular lens 1 is to be placed (held). The inserting portion 11 is a tube-shaped part tapered at the front end, in which the intraocular lens 1 folded at the lens place portion 12 passes through a hollow portion and is folded smaller, and then extruded out (discharged outside) through an exit (an outlet) 11a. The lens place portion 12 includes a fixed part 12a that is fixed to the inserting portion 11 and a movable part (an opening/closing part) 12b that is coupled to the fixed part 12a through a hinge 13. The fixed part 12a includes a placing surface 14a inside and the movable part 12b includes a placing surface 14b inside. The intraocular lens 1 is placed on the placing surfaces 14a and 14b in a state that the lens place portion 12 (the fixed part 12a and the movable part 12b) is opened. The intraocular lens 1 placed in the state that the lens place portion 12 is opened is not folded (but, movement in the opening/closing direction of the lens place portion 12 is restricted). When the lens place portion 12 (the fixed part 12a and the movable part 12b) is closed after the intraocular lens 1 is placed, the placing surfaces 14a and 14b form the same shape (half circle) as an inlet 11b of the inserting portion 11 and the intraocular lens 1 placed on the placing surfaces 14a and 14b is folded in the same shape. In other word, the placing surfaces 14a and 14b are curved such that the intraocular lens 1 is not folded when the lens place portion 12 is opened, and it is folded when the lens place portion 12 is closed.
Further, when the lens place portion 12 is opened, a space 19 is defined by a sidewall 120a of the fixed part 12a facing the movable part 12b and a sidewall 120b of the movable part 12b facing the fixed part 12a. With the lens place portion 12 open, the center of the optical portion 2 of the intraocular lens 1 placed on the lens place portion 12 is positioned above the space 19.

The fixed part 12a is provided with a cover 15a and the movable part 12b is provided with a cover 15b. The covers 15a and 15b cover the upside of the placing surfaces 14a and 14b when the lens place portion 12 is closed. A convex portion 16 extending down toward the placing surfaces 14a and 14b is formed at the side of the cover 15b facing the cover 15a . The convex portion 16 prevents the intraocular lens 1 from being folded in directions, not the appropriate folding direction of the intraocular lens 1, i.e. not the opening/closing direction of the lens place portion 12.

Further, a joint portion 17a extending upward is provided at the side of the cover 15a facing the cover 15b and a joint portion 17b extending upward is provided at the side of the cover 15b facing the cover 15a. The joint portion 17a is provided with a female hook 170a that is engaged (snap-fitted) with a male hook 170b provided to the joint portion 17b. When the lens place portion 12 (the fixed part 12a and the movable part 12b) is closed, the joint portion 17a is jointed with the joint portion 17b and the female hook 170a is engaged with the male hook 170b.
A convex fitting portion 18 is provided at an outside of the fixed part 12a (described hereinafter).

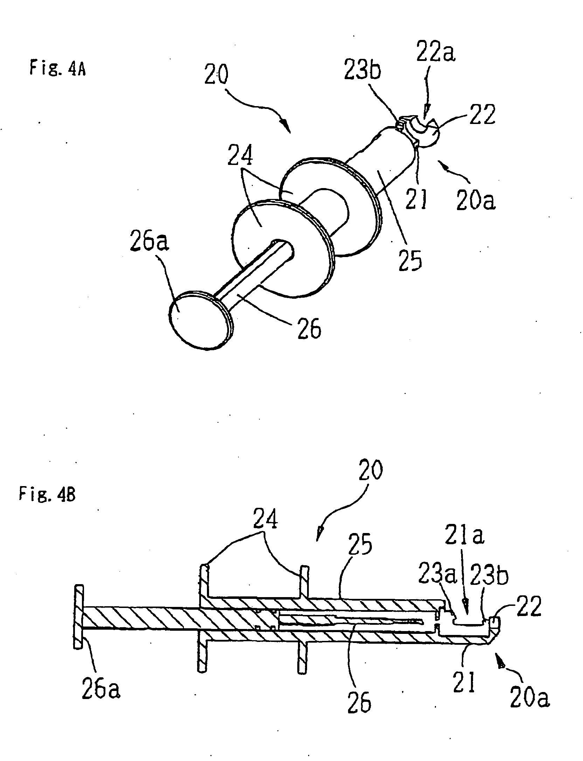

Figs. 4A and 4B are schematic views of the handpiece 20. Fig. 4A is a perspective view and Fig. 4B is a cross-sectional view. The handpiece 20 includes a cylinder (a body unit) 25 where the cartridge 10 is mounted and a piston (a lens extruding unit) 26 that is inserted in the cylinder 25. A mounting portion 20a for the cartridge 10 is formed at the front end of the cylinder 25. Further, a flare-shaped finger portion 24 where a user (an operator) puts his/her fingers to hold the handpiece 20 (the cylinder 25) such as a syringe is formed on the outer periphery of the cylinder 25. A pushing portion 26a for the thumb to push the piston 26 into the cylinder 25 is provided at the back end of the piston 26.

The mounting portion 20a includes a first receiving portion 21 for the lens place portion 12 (the fixed part 12a) of the cartridge 10 to be placed (fitted) and a second receiving portion 22 for the inserting portion 11 of the cartridge 10 to be placed (fitted). The first receiving portion 21 is shaped like a quadrant circle at the end of the cylinder 25 and the second receiving portion 22 has a letter-C shape in which an arc thereof is a little longer than a half of the circumference around the center axis of the cylinder 25. When the cartridge 10 is mounted on the mounting portion 20a, the bottom of the fixed part 12a contacts with the first receiving portion 21 and the bottom of the inserting portion 11 contacts with the second receiving portion 22. A recess 21a is formed on the first receiving portion 21 by two convex portions 23a and 23b and the fitting portion 18 (see Fig. 3) of the cartridge 10 is fitted in the recess 21a. Further, the inserting portion 11 is fitted in a recess 22a of the second receiving portion 22. As combined as described above, the cartridge 10 is fixedly held at the mounting portion 20a in the state that the lens place portion 12 (the fixed part 12a and the movable part 12b) is opened.

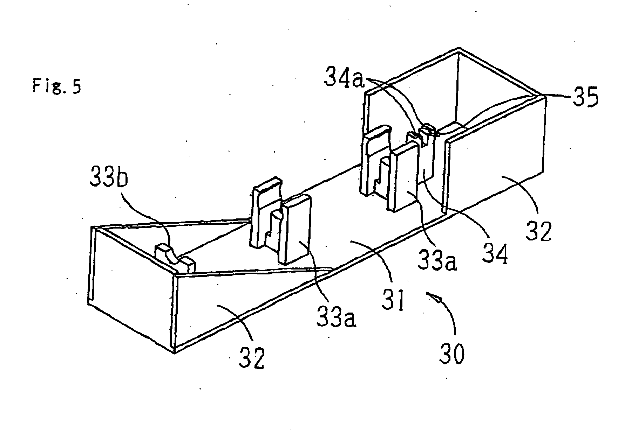

Fig. 5 is a schematic view of the holder 30. A base 31 is provided with a protective wall 32 (a fence) along its edge and includes holding portions 33a and 33b for holding the injector 50. Two holding portions 33a are provided so as to chuck the cylinder 25 and the holding portion 33b supports the piston 26 from underneath. A circular arc spot facing for the cylinder 25 fitted is formed at the upside of the holding portion 33a and another circular arc spot facing for the piston 26 fitted is formed at the upside of the holding portion 33b.

The base 31 is provided with a restrictor 34 that restricts movement of the intraocular lens 1 in a forward/backward direction of the injector 50 (a perpendicular direction to the opening/closing direction of the lens place portion 12) in the lens place portion 12 of the cartridge 10. The restrictor 34 extends in the direction that the injector 50 is detached from the holder 30. A cavity 35 where the intraocular lens 1 (the optical portion 2) is placed is formed on upside of the restrictor 34. The front and back sidewalls 34a of the cavity 35 are curved to fit with the circular optical portion 2 and chuck the optical portion 2 from front and rear when the optical portion 2 is placed in the cavity 35. Further, the bottom of the cavity 35 is more curved than the optical portion 2, so that when the optical portion 2 is placed in the cavity 35, the periphery of the optical portion 2 contacts with the edge inside the cavity 35. Accordingly, the intraocular lens 1 is stably placed (held).
Meanwhile, the width of the restrictor 34 in a left/right direction (the opening/closing direction of the lens place portion 12) is smaller than the diameter of the optical portion 2, so that a part of the edge of the optical portion 2 protrudes to the left and right of the cavity 35.

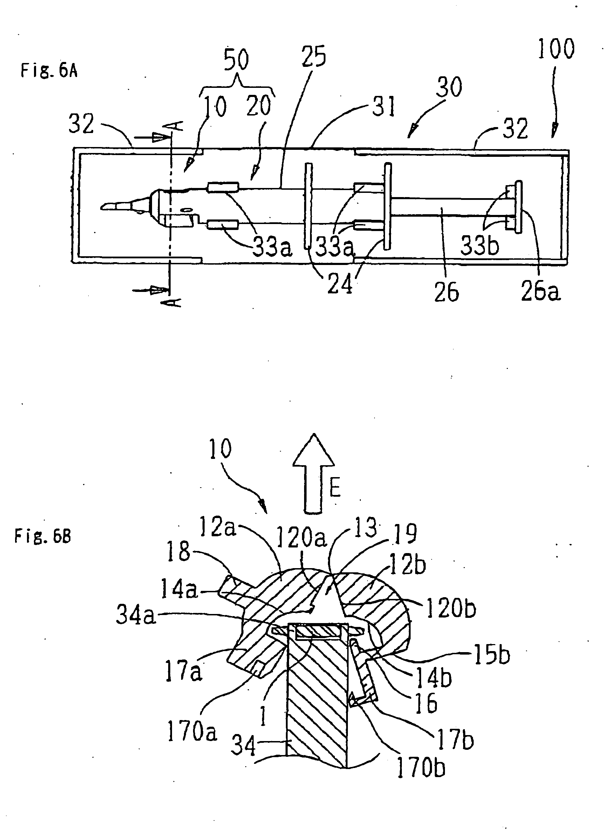

Figs. 6A and 6B are views showing a state in which the injector 50 is held by the holder 30 (i.e. an intraocular lens injecting system 100). Fig. 6A is a plan view taken from above and Fig. 6B is a cross-sectional view taken along a line A-A of Fig. 6A. The lens place portion 12 of the cartridge 10 is opened and the intraocular lens 1 placed in the cavity 35 of the restrictor 34 is covered by the cartridge 10. On the other hand, movement of the piston 26 in the axial direction thereof (the forward/backward direction) is restricted by the holding portion 33b. In the above state, because the front end of the piston 26 is not inserted in the cartridge 10, the intraocular lens 1 is not extruded out by the piston 26,
Fig. 6B is a view showing an arrangement state of the intraocular lens 1 within the injector 50 (the cartridge 10) held by the holder 30. The intraocular lens 1 (the optical portion 2) is placed on the restrictor 34 and covered by the cartridge 10 such that it does not contact with the placing surfaces 14a and 14b in the lens place portion 12. As described above, because the width in the left/right direction of the restrictor 34 is smaller than the diameter of the optical portion 2 and a part of the edge of the optical portion 2 protrudes to the left and right of the restrictor 34, when the injector 50 (the cartridge 10) is detached from the holder 30 (the holding portions 33a and 33b) in the direction of an arrow E, the intraocular lens 1 is caught by the placing surfaces 14a and 14b of the cartridge 10 and remains in the lens place portion 12.

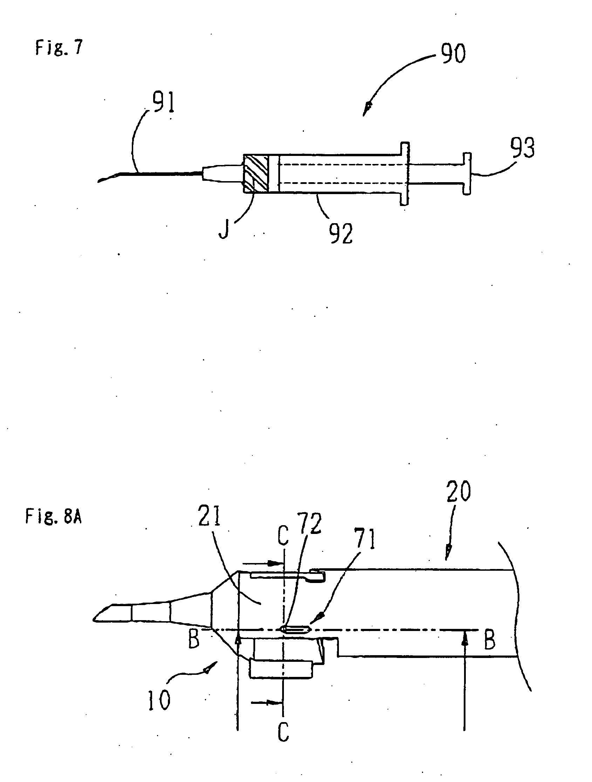

FIG. 7 is a perspective view of a syringe 90 to inject lubricant of hialuronan natrium etc. into the cartridge 10. The syringe 90 includes an injecting needle 91 with the front end curved, a cylinder 92 to receive lubricant J, and a piston 93.

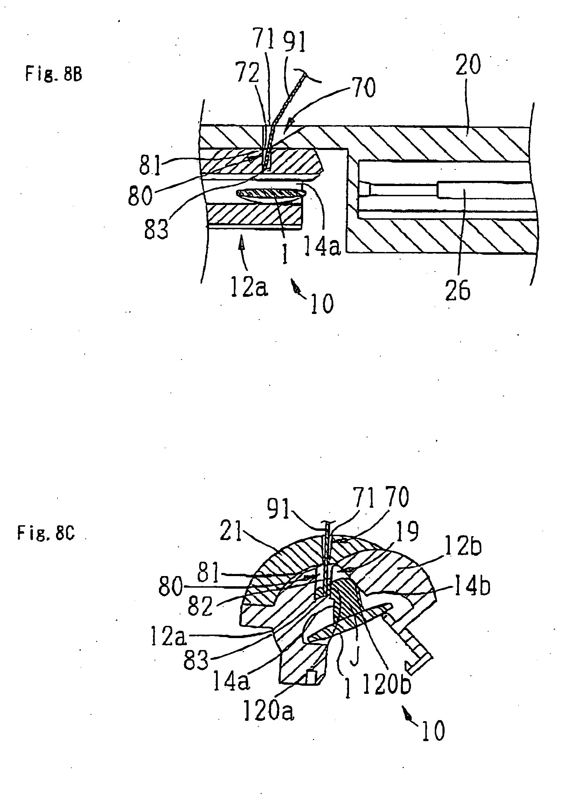

FIGS. 8A, 8B, and 8C are views illustrating a configuration for injecting the lubricant J into the cartridge 10. FIG. 8A is a view of the injector 50 supported by the holder 30 seen from the above, FIG. 8B is a cross-sectional view of FIG. 8A taken along a line B-B, and FIG. 8C is a cross-sectional view of FIG. 8A taken along a line C-C.

An injecting passage (an injection hole) 80 for injecting the lubricant J into the lens place portion 12 is formed in the cartridge 10. The injecting passage 80 is formed through the outerwall of the fixed part 12a to the sidewall 120a of the fixed part 12a. That is, the injecting passage 80 has an inlet 81 at the outerwall of the fixed part 12a and an outlet 82 at the sidewall 120a of the fixed part 12a. The outlet 82 is formed at the sidewall 120a such that it is positioned at the vicinity of the center of the optical portion 2 of the intraocular lens 1 placed in the state that the lens place portion 12 open (see FIG. 8B).

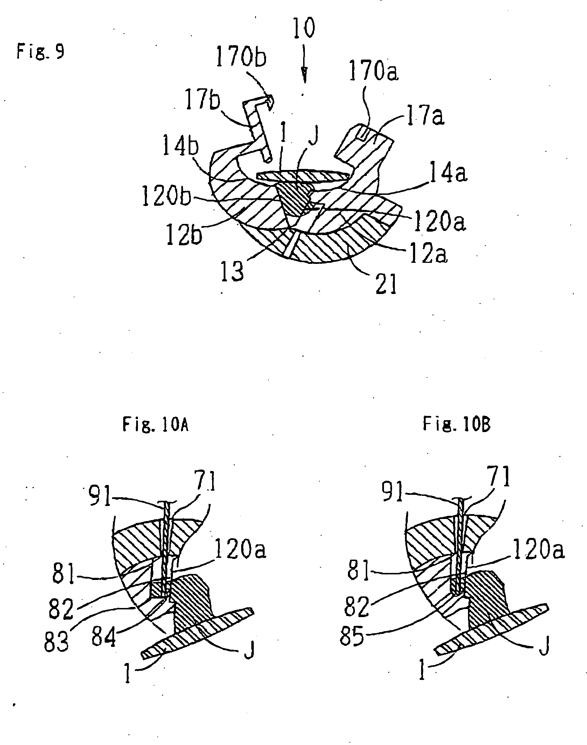

Further, an injecting passage (an injection hole) 70 for injection the lubricant J into the cartridge 10 (the lens place portion 12) is formed in the first receiving portion 21 of the handpiece 20. The injecting passage 70 is formed through the outerwall to the innerwall at substantially center of the first receiving portion 21-That is, the injecting passage 70 has an inlet 71 at the outerwall of the first receiving portion 21 and an outlet 72 at the innerwall of the first receiving portion 21.
The inlet 71 is longer than the outlet 72 in the forward/backward direction (see FIG- 8) and the injecting passage 70 is tapered forward (see FIG. 8B).
When the cartridge 10 is mounted on the mounting portion 20a of the handpiece 20, the outlet 72 of the injecting passage 70 is communicated with the inlet 81 of the injecting passage 80. As the injecting needle 91 of the syringe 90 is inserted into the inlet 71, the injecting needle 91 is inserted into the inlet 81 through the outlet 72 and contacts with an innerwall 83 of the injecting passage 80. Accordingly, when the lubricant J is injected into the cartridge 10 (the lens place portion 12) by the syringe 90, the movement of the injecting needle 91 is restricted, so that damage in the intraocular lens 1 due to the contact of the front end of the injecting needle 91 is prevented. Further, the innerwall 83 is inclined at a predetermined angle to the insert direction of the injecting needle 91 (see FIG. 8C), so that it prevents the injecting needle 91 from protruding inside the space 19 through the outlet 82. Accordingly, the front end of the injecting needle 91 does not contact with the intraocular lens 1.
The configuration for restricting the movement of the injecting needle 91 is not limited to that shown in FIG. 8C, and may be modified in a variety of ways. For example, as shown in FIG. 10A, a convex portion 84 may be formed at the outlet 82 in the innerwall 83. Further, as shown in FIG. 10B, a concave portion 85 may be formed on the innerwall 83. A configuration for restricting the movement of the injecting needle 91 may be formed in the syringe 90. For example, a block may be provided at a predetermined position of the injecting needle 91, and the movement of the injecting needle 91 is restricted by contacting the block with the first receiving portion 21.

The operation of the above injecting system 100 is now described in detail. First, a user (an operator) takes out the injecting system 100 from a case (not shown) for maintaining an aseptic condition and detaches the injector 50 (the cartridge 10) from the holder 30. In this operation, the cartridge 10 is moved in the direction of the arrow E (above the holder 30). However, the intraocular lens 1 placed on the restrictor 34 is not restricted in movement in the detaching direction, so that it can be lifted in contact with the placing surfaces 14a and 14b and is detached from the restrictor 34 (the cavity 35) (that is, released from the restriction by the restrictor 34), and in turn remains in the cartridge 10. In other words, before the lubricant J is injected (described later), the intraocular lens 1 is placed (held) in advance in the lens place portion 12.

The injection of the lubricant J into the cartridge 10 (the lens place portion 12) will be now described. The injecting needle 91 of the syringe 90 receiving the lubricant J is first inserted through the inlet 71 until the needle 91 contacts with the innerwall 83. Subsequently, the lubricant J is discharged through the front end of the injecting needle 91 by pushing the piston 93. The discharged lubricant J flows out through the outlet 82 and collects on the intraocular lens 1 (see FIG. 8C).

The lubricant J is injected into the cartridge 10 (the lens place portion 12) in a predetermined amount, and the injector 50 (the cartridge 10) rotates to direct the hinge 13 down. Accordingly, the lubricant J collects in the space 19 (see FIG. 9). As the lens place portion 12 (the fixed part 12a and the movable part 12b) is closed and the space correspondingly narrows, the lubricant J collecting in the space 19 gradually flows into a space between the placing surfaces 14a and 14b and the intraocular lens 1. When the lens place portion 12 (the fixed part 12a and the movable part 12b) is completely closed, the space 19 does not exist any more and the lubricant J remains between the placing surfaces 14a and 14b and the intraocular lens 1. Accordingly, the lubricant J is inserted into the cartridge 10 (the lens place portion 12) in the state that the intraocular lens 1 is placed (held) inside in advance.

With the lens placing portions 12 (the fixed part 12a and the movable part 12b) closed, the intraocular lens 1 is bent by the placing surfaces 14a and 14b that pushing the intraocular lens 1. When starting moving forward by pushing the pushing portion 26a, the piston 26 moves inside the cylinder 25 to the front end and pushes the intraocular lens 1 from the lens placing portion 12 into the inserting portion 11. As pushed into the inserting portion 11, the intraocular lens 1 is bent small (rounded). Accordingly, the intraocular lens 1 bent small is discharged through the outlet 11a at the front end of the inserting portion 11.

According to the present embodiment, although an injector having a lens cartridge integrally formed with a handpiece and held by a holder is provided, it is not limited thereto. In other words, only a lens cartridge may be held by a holder. Further, if the lens cartridge is not covered by the hand piece (the first receiving portion 21 in the present embodiment), it is not needed to form the injecting passage (the injecting passage 70 in the present embodiment) in the handpiece.
Further, according to the invention, since the injector is held by the holder, the intraocular lens is placed (held) on a part of the holder (the restrictor 34 in the present invention), but, not limited thereto, may be placed (held) in the lens cartridge (e.g. the lens place portion 12).

## Claims

1. An intraocular lens injector (50) for injecting a foldable intraocular lens (1) in an eye, the injector comprising:
a lens cartridge (10) including
an inserting portion (11) that includes an outlet (11a) for the intraocular lens and is insertable through an incision made on the eye to inject the lens in the eye; and
a lens place portion (12) where the intraocular lens is placed and which is formed on an opposite side to the outlet of the inserting portion, wherein the lens place portion can be closed through a hinge (13) in a direction in which the intraocular lens is folded and the placed intraocular lens is folded by closing the lens place portion; and
a hand piece (20) to which the lens cartridge is mounted,
**characterized in that**
a first injecting passage (80) is formed in the lens place portion to inject lubricant (J) into the lens place portion from an outside, and
the hand piece includes a second injecting passage (70) for injecting lubricant into the lens place portion from the outside, the second injectingpassage communicating with the first infecting passage when the lens cartridge is mounted to the hand piece.

2. The intraocular lens injector according to claim 1, wherein
the lens place portion includes a fixed part (12a) fixed to the inserting portion and a movable part (12b) connected to the fixed part through the hinge, and
the lubricant (J) is injected into a space between the fixed and movable parts in a state that the fixed and movable parts are opened.

3. The intraocular lens injector according to claim 2, wherein the first injecting passage is formed in the fixed part so that an inlet (81) is formed at an outerwall of the fixed part and an outlet (82) is formed at a sidewall of the fixed part facing the movable part.

4. The intraocular lens injector according to any one of claims 1 through 3, wherein the first injecting passage includes a restricting portion (83, 84, and 85) for restricting movement of an injecting needle (91) of a syringe (90) for injecting the lubricant.

5. An intraocular lens injecting system comprising a holder (30) that holds an intraocular lens injector according to any one of claims 1 to 4.

6. The intraocular lens injecting system according to claim 5, wherein
the holder includes a holding member (34, 34a, and 35) that holds the intraocular lens in the open lens place portion, and
the lubricant is injected into the open lens place portion.

## Patentansprüche

1. Injektor (50) für Intraokularlinsen zum Einführen einer Intraokular-Faltlinse (1) in ein Auge, wobei der Injektor aufweist:
- eine Linsenkassette (10), umfassend:
- einen Einsetzbereich (11), der einen Auslass (11a) für die Intraokularlinse umfasst und durch einen am Auge durchgeführten Schnitt einsetzbar ist, um die Linse in das Auge einzuführen; und
- einen Linsenanordnungsbereich (12), wo die Intraokularlinse angeordnet ist, und der auf einer gegenüberliegenden Seite des Auslasses des Einsetzbereichs ausgebildet ist, wobei der Linsenanordnungsbereich durch ein Gelenk (13) in eine Richtung geschlossen werden kann, in die die Intraokularlinse gefaltet ist und die angeordnete Intraokularlinse durch Schließen des Linsenanordnungsbereich gefaltet wird; und
- ein Handstück (20), an dem die Linsenkassette befestigt ist, **dadurch gekennzeichnet, dass**
ein erster Einspritzkanal (80) im Linsenanordnungsbereich ausgebildet ist, um Gleitmittel (J) in den Linsenanordnungsbereich von außen einzuspritzen, und
das Handstück einen zweiten Einspritzkanal (70) zum Einspritzen von Gleitmittel in den Linsenanordnungsbereich von außen umfasst, wobei der zweite Einspritzkanal mit dem ersten Einspritzkanal zusammenwirkt, wenn die Linsenkassette am Handstück befestigt ist.

2. Injektor für Intraokularlinsen gemäß Anspruch 1, wobei der Linsenanordnungsbereich ein fixiertes Teil (12a), das am Einsetzbereich fixiert ist, und ein bewegliches Teil (12b) umfasst, das mit dem fixierten Teil durch das Gelenk verbunden ist, und
das Gleitmittel (J) in einen Raum zwischen dem fixierten und beweglichen Teil in einem Zustand eingespritzt wird, bei dem die fixierten und beweglichen Teile geöffnet sind.

3. Injektor für Intraokularlinsen gemäß Anspruch 2, wobei der erste Einspritzkanal im fixierten Teil ausgebildet ist, so dass ein Einlass (81) an einer Außenwand des fixierten Teils und ein Auslass (82) an einer Seitenwand des fixierten Teils, das dem beweglichen Teil zugewandt, ausgebildet ist.

4. Injektor für Intraokularlinsen gemäß einem der Ansprüche 1 bis 3, wobei der erste Einspritzkanal einen begrenzenden Bereich (83, 84, 85) zum Begrenzen einer Bewegung einer Einspritznadel (91) einer Spritze (90) zum Einspritzen des Gleitmittels umfasst.

5. Intraokularlinsen-Einspritzsystem mit einer Halterung (30), die einen Injektor für Intraokularlinsen gemäß einem der Ansprüche 1 bis 4 hält.

6. Intraokularlinsen-Einspritzsystem gemäß Anspruch 5, wobei
- die Halterung ein Halteelement (34, 34a, 35) umfasst, das die Intraokularlinse im geöffneten Linsenanordnungsbereich hält, und
- das Gleitmittel in den geöffneten Linsenanordnungsbereich eingespritzt wird.

## Revendications

1. Injecteur pour lentille intraoculaire (50) pour injecter une lentille intraoculaire pliable (1) dans un oeil, l'injecteur comprenant :
une cartouche de lentille (10) comprenant
une partie d'insertion (11) qui comprend une sortie (11a) pour la lentille intraoculaire insérable par une incision pratiquée sur l'oeil pour injecter la lentille dans l'oeil : et
une partie de placement de la lentille (12) où la lentille intraoculaire est placée et qui est formée sur un côté opposé à la sortie de la partie d'insertion, la partie de placement de la lentille pouvant être fermée par une charnière (13) dans une direction dans laquelle la lentille intraoculaire est repliée et la lentille intraoculaire placée est pliée en fermant la partie de placement de la lentille : et
une pièce à main (20) sur laquelle la cartouche de lentille est montée,
**caractérisé en ce que**
un premier passage d'injection (80) est formé dans la partie de placement de lentille pour injecter un lubrifiant (J) dans la partie de placement de lentille de l'extérieur, et
la pièce à main comprend un deuxième passage d'injection (70) pour injecter un lubrifiant dans la partie de placement de lentille de l'extérieur, le deuxième passage d'injection communiquant avec le premier passage d'injection lorsque la cartouche de lentille est montée sur la pièce à main.

2. Injecteur de lentille intraoculaire selon la revendication 1, dans lequel la partie de placement de lentille comprend une partie fixe (12a) fixée à la partie d'insertion et une partie mobile (12b) reliée à la partie fixe par la charnière, et
le lubrifiant (J) est injecté dans un espace entre les parties fixe et mobile de telle sorte que les parties fixe et mobile soient ouvertes.

3. Injecteur de lentille intraoculaire selon la revendication 2, dans lequel le premier passage d'injection est formé dans la partie fixe de sorte qu'une entrée (81) soit formée sur une paroi extérieure de la partie fixe et qu'une sortie (82) soit formée sur une paroi latérale de la partie fixe faisant face à la partie mobile.

4. Injecteur de lentille intraoculaire selon l'une quelconque des revendications 1 à 3, dans lequel le premier passage d'injection comprend une partie de restriction (83, 84 et 85) pour restreindre le mouvement d'une aiguille d'injection (91) d'une seringue (90) destinée à injecter le lubrifiant.

5. Système d'injection de lentille intraoculaire, comprenant un support (30) qui soutient un injecteur de lentille intraoculaire selon l'une quelconque des revendications 1 à 4.

6. Système d'injection de lentille intraoculaire selon la revendication 5, dans lequel
le support comprend un élément de support (34, 34a et 35) qui soutient la lentille intraoculaire dans la partie de placement de lentille ouverte et le lubrifiant est injecté dans la partie de placement de lentille ouverte.
